# EUROPEAN PATENT APPLICATION

(11) **EP 4 559 425 A1**
(43) Date of publication of application: **28.05.2025**
(21) Application number: 24212821.3
(22) Date of filing: 13.11.2024
(51) Int. Cl.: A61B 34/20

(54) **SYSTEMS FOR POINT AND TOOL ACTIVATION**

(30) Priority: 27.11.2023 US 202363602911 P; 27.11.2023 US 202363602866 P
(71) Applicant: Smith & Nephew, Inc., Memphis, TN 38116 (US); Smith & Nephew Orthopaedics AG, 6300 Zug (CH); Smith & Nephew Asia Pacific Pte. Limited, Singapore 138565 (SG)
(72) Inventor: QUIST, Brian William, Salem, 03079 (US); MELO TEIXEIRA, Rui Jorge, 3460-613 Tondela (PT); DI STEFANO, Craig Anthony, Andover, 01810 (US)
(74) Representative: Mathys & Squire

(57) **Abstract**

A method for dynamically assigning actions to a plurality of input devices of a first surgical instrument connected to a surgical system includes, using one or more computing devices, with the surgical system in a first state, assigning a first action to a first input device of the plurality of input devices. In the first state, the surgical system is configured to cause the first action to be performed in response to the first input device being actuated. The method further includes detecting a second surgical instrument and, in response to detecting the second surgical instrument, transitioning the surgical system to a second state and assigning a second action to the first input device. In the second state, the surgical system is configured to cause the second action to be performed in response to the first input device being actuated.

## Description

### FIELD

The present disclosure relates to probes for surgical systems, and more particularly to probes for registering points of an anatomical site for a surgical procedure, capturing images, and/or performing other functions.

### BACKGROUND

The background description provided here is for the purpose of generally presenting the context of the disclosure. Work of the presently named inventors, to the extent it is described in this background section, as well as aspects of the description that may not otherwise qualify as prior art at the time of filing, are neither expressly nor impliedly admitted as prior art against the present disclosure.

Arthroscopic surgical procedures are minimally invasive surgical procedures in which access to the surgical site within the body is by way of small keyholes or ports through the patient's skin. The various tissues within the surgical site are visualized by way of an arthroscope placed through a port, and the internal scene is shown on an external display device. The tissue may be repaired or replaced through the same or additional ports. In computer-assisted surgical procedures (e.g., replacement of the anterior cruciate ligament (ACL), reduction of femora-acetabular impingement), the location of various objects with the surgical site may be tracked relative to the bone by way of images captured by an arthroscope and a three-dimensional model of the bone.

### SUMMARY

Aspects of the invention are as set out in the appended claims.

A method for dynamically assigning actions to a plurality of input devices of a first surgical instrument connected to a surgical system includes, using one or more computing devices, with the surgical system in a first state, assigning a first action to a first input device of the plurality of input devices. In the first state, the surgical system is configured to cause the first action to be performed in response to the first input device being actuated. The method further includes detecting a second surgical instrument and, in response to detecting the second surgical instrument, transitioning the surgical system to a second state and assigning a second action to the first input device. In the second state, the surgical system is configured to cause the second action to be performed in response to the first input device being actuated.

In other features, the first state and the second state correspond to a state of an application being executed using the surgical system. The first surgical instrument includes a camera head, and the plurality of input devices includes a plurality of buttons arranged on the camera head. The surgical system includes at least one of a programmable footswitch with configurable buttons and a system configured to capture gestures as inputs. The second surgical instrument includes at least one of an aimer, an aligner, a probe, a grasper, a pincher, a scissor, a punch, a microfracture pick, a knife, a meniscectomy tool, a curette, an anchor deployment device, a biologic deployment device, a suture anchor deployment device, an arthroscopic blade, an arthroscopic RF wand, an arthroscopic coblation wand, a bone fiducial, and a fiducial on an instrument. The plurality of input devices includes a second input device, and wherein a third action assigned to the second input device is the same regardless of whether the surgical system is in the first state or the second state. Detecting the second surgical instrument includes detecting the second surgical instrument using a camera.

In other features, the method further includes transitioning the surgical system to the second state in response completion of a current action by one or more of the surgical system, the first surgical instrument, and the second surgical instrument. The method further includes detecting a bone fiducial prior to detecting the second surgical instrument. The first surgical instrument is an arthroscopic instrument including a camera head, and the bone fiducial is configured to be fixedly attached to patient anatomy. The method further includes, in response to detecting the bone fiducial, transitioning the surgical system to the first state and assigning the first action to the first input device and, in response to detecting the second surgical instrument, transitioning the surgical system to the second state and assigning the second action to the first input device.

A processor is configured to execute instructions stored in memory to dynamically assign actions to a plurality of input devices of a first surgical instrument connected to a surgical system. Executing the instructions causes the processor to, with the surgical system in a first state, assign a first action to a first input device of the plurality of input devices. In the first state, the surgical system is configured to cause the first action to be performed in response to the first input device being actuated. The instructions further cause the processor to detect a second surgical instrument and, in response to detecting the second surgical instrument, transition the surgical system to a second state and assign a second action to the first input device. In the second state, the surgical system is configured to cause the second action to be performed in response to the first input device being actuated.

In other features, the first state and the second state correspond to a state of an application being executed using the surgical system. The first surgical instrument includes a camera head, and the plurality of input devices includes a plurality of buttons arranged on the camera head. The processor is configured to receive inputs from at least one of a programmable footswitch with configurable buttons and a system configured to capture gestures as the inputs. The second surgical instrument includes at least one of an aimer, an aligner, a probe, a grasper, a pincher, a scissor, a punch, a microfracture pick, a knife, a meniscectomy tool, a curette, an anchor deployment device, a biologic deployment device, a suture anchor deployment device, an arthroscopic blade, an arthroscopic RF wand, an arthroscopic coblation wand, a bone fiducial, and a fiducial on an instrument. The plurality of input devices includes a second input device, and a third action assigned to the second input device is the same regardless of whether the surgical system is in the first state or the second state. Detecting the second surgical instrument includes detecting the second surgical instrument using a camera.

In other features, executing the instructions further causes the processor to transition the surgical system to the second state in response completion of a current action by one or more of the surgical system, the first surgical instrument, and the second surgical instrument. Executing the instructions further causes the processor to detect a bone fiducial prior to detecting the second surgical instrument. The first surgical instrument is an arthroscopic instrument including a camera head, and wherein the bone fiducial is configured to be fixedly attached to patient anatomy. Executing the instructions further causes the processor to, in response to detecting the bone fiducial, transition the surgical system to the first state and assign the first action to the first input device and, in response to detecting the second surgical instrument, transition the surgical system to the second state and assign the second action to the first input device.

A method for operating a surgical instrument coupled to a surgical system includes, using one or more computing devices, detecting, via a connection interface defined on a handpiece of the surgical instrument, a first device type of a first surgical device coupled to the handpiece, in response to detecting the first device type of the first surgical device, assigning first actions to respective input devices of a plurality of input devices arranged on the handpiece, and, in response to actuation of one or more of the plurality of input devices, controlling at least one of the surgical instrument and the surgical system in accordance with the first actions assigned to the plurality of input devices.

In other features, detecting the first device type includes at least one of detecting the first device type based on an electrical signal or input data received from the first surgical device, detecting the first device type based on mechanical engagement between the first surgical device and the connection interface, and detecting a magnetic signal between the first surgical device and the connection interface. The first device type corresponds to a probe device configured to interact with an anatomical surface for a surgical operation. The method further includes detecting, via the connection interface defined on a handpiece of the surgical instrument, a second device type of a second surgical device coupled to the handpiece, in response to detecting the second device type of the second surgical device, assigning second actions to the respective input devices of the plurality of input devices arranged on the handpiece, and, in response to actuation of the one or more of the plurality of input devices, controlling at least one of the surgical instrument and the surgical system in accordance with the second actions assigned to the plurality of input devices. The second device type corresponds to at least one of a shaver blade and a burr tool. The first actions correspond to registration functions for transmitting registration inputs from the surgical instrument to the surgical system and the second actions correspond to motor control functions for controlling a shaving or cutting operation of the surgical instrument.

In other aspects, a system is configured to perform functions corresponding to various methods described herein. In other aspects, a processor is configured to execute instructions stored in memory to perform functions of various methods described herein.

Further areas of applicability of the present disclosure will become apparent from the detailed description, the claims and the drawings. The detailed description and specific examples are intended for purposes of illustration only and are not intended to limit the scope of the disclosure.

### BRIEF DESCRIPTION OF THE DRAWINGS

For a detailed description of example embodiments, reference will now be made to the accompanying drawings in which:
Figure 1 shows a surgical system in accordance with at least some embodiments;
Figure 2 shows a conceptual drawing of a surgical site with various objects within the surgical site tracked, in accordance with at least some embodiments;
Figure 3 shows a method in accordance with at least some embodiments;
Figure 4 is an example video display showing portions of a femur and a bone fiducial during a registration procedure, in accordance with at least some embodiments;
Figure 5 shows a method in accordance with at least some embodiments;
Figure 6 shows an example method for progressing between various system and/or application states in accordance with at least some embodiments;
Figure 7 shows an example instrument configured to have fixed or variable states in accordance with different application states in accordance with at least some embodiments;
Figures 8A and 8B show another example implementation of an instrument configured to connect to different device/tool types in accordance with at least some embodiments;
Figure 9 shows an example method for controlling an instrument configured to connect to different device/tool types in accordance with at least some embodiments; and
Figure 10 shows an example computer system or computing device configured to implement the various systems and methods of the present disclosure.

In the drawings, reference numbers may be reused to identify similar and/or identical elements.

### DEFINITIONS

Various terms are used to refer to particular system components. Different companies may refer to a component by different names - this document does not intend to distinguish between components that differ in name but not function. In the following discussion and in the claims, the terms "including" and "comprising" are used in an openended fashion, and thus should be interpreted to mean "including, but not limited to...." Also, the term "couple" or "couples" is intended to mean either an indirect or direct connection. Thus, if a first device couples to a second device, that connection may be through a direct connection or through an indirect connection via other devices and connections.

"A", "an", and "the" as used herein refers to both singular and plural referents unless the context clearly dictates otherwise. By way of example, "a processor" programmed to perform various functions refers to one processor programmed to perform each and every function, or more than one processor collectively programmed to perform each of the various functions. To be clear, an initial reference to "a [referent]", and then a later reference for antecedent basis purposes to "the [referent]", shall not obviate the fact the recited referent may be plural.

The terms "input" and "output" when used as nouns refer to connections (e.g., electrical, software) and/or signals, and shall not be read as verbs requiring action. For example, a timer circuit may define a clock output. The example timer circuit may create or drive a clock signal on the clock output. In systems implemented directly in hardware (e.g., on a semiconductor substrate), these "inputs" and "outputs" define electrical connections and/or signals transmitted or received by those connections. In systems implemented in software, these "inputs" and "outputs" define parameters read by or written by, respectively, the instructions implementing the function. In examples where used in the context of user input, "input" may refer to actions of a user, interactions with input devices or interfaces by the user, etc.

"Controller," "module," or "circuitry" shall mean, alone or in combination, individual circuit components, an application specific integrated circuit (ASIC), a microcontroller with controlling software, a reduced-instruction-set computer (RISC) with controlling software, a digital signal processor (DSP), a processor with controlling software, a programmable logic device (PLD), a field programmable gate array (FPGA), or a programmable system-on-a-chip (PSOC), configured to read inputs and drive outputs responsive to the inputs.

As used to describe various surgical instruments or devices, such as a probe, the term "proximal" refers to a point or direction nearest a handle of the probe (e.g., a direction opposite the probe tip). Conversely, the term "distal" refers to a point or direction nearest the probe tip (e.g., a direction opposite the handle).

### DETAILED DESCRIPTION

The following discussion is directed to various embodiments of the invention. Although one or more of these embodiments may be preferred, the embodiments disclosed should not be interpreted, or otherwise used, as limiting the scope of the disclosure, including the claims. In addition, one skilled in the art will understand that the following description has broad application, and the discussion of any embodiment is meant only to be exemplary of that embodiment, and not intended to intimate that the scope of the disclosure, including the claims, is limited to that embodiment.

Surgical procedures may implement various systems and methods for operating and controlling surgical systems, such as arthroscopic video-based navigation systems and associated surgical tools or instruments. For example, navigation systems and tools (e.g., probes) may be used for registering a three-dimensional model of a rigid structure, such as bone, capturing images, and so on. In some examples, these systems are configured to identify surface features of a rigid structure visible in a video stream, and use the surface features to register a three-dimensional model for use in computer-assisted navigation of the surgical procedure. In some examples, the surface features are determined using touchless techniques based on a known or calculated motion of the camera. In other examples, the surface features are gathered using a touch probe that is not itself directly tracked; rather, the pose of the touch probe, and thus the locations of the distal tip of the touch probe touching the bone, may be determined by segmenting the frames of the video stream and pose estimation. In yet still further examples, the three-dimensional model may be registered by use of a patient-specific instrument that couples to the rigid structure in only one orientation; thus, a fiducial coupled to the patient-specific instrument, or in some cases the patient-specific instrument itself without a fiducial, may be used to register the three-dimensional bone model.

Various examples are described herein in the context of anterior-cruciate ligament (ACL) repair (e.g., for placing femoral and/or tibial tunnels during ACL reconstruction). In this context, the rigid structure is bone, and the three-dimensional mode is a three-dimensional bone model. However, the techniques are applicable to any suitable rigid anatomical structure. Moreover, the various techniques may be applicable to many types of surgical procedures, such as repairs associated with the knee, the hip, the shoulder, the wrist, or the ankle. Thus, the description and developmental context shall not be read as a limitation of the applicability of the teachings.

In some examples of a registration procedure, a user (e.g., a surgeon) probes an anatomical surface using a handheld probe. Collected points (e.g., a point cloud) are processed (e.g., using a machine learning algorithm) and matched to a bone model, such as a bone model created via a scan (e.g., a CT or MRI scan) or other technique. For example, the bone model is overlaid on top of a live arthroscopic video feed to provide an augmented or mixed reality visual representation of a surgical or anatomical site.

One challenge associated with these navigation systems is determining when a user intends for a particular action or function (e.g., such as point acquisition or other registration functions, capturing images, etc.) to be performed. For example, during conventional implementations of a registration procedure, the user collects points by manual inputting commands to the surgical system to start, pause, and end collection using a tablet or other computing device (e.g., as controlled by nursing or other surgical staff), arthroscope camera head buttons, etc. As another example, navigation systems may require user prompting or inputs to advance application states, enable different functions, enable different instruments, etc.

Systems and methods of the present disclosure are configured to facilitate functions related to informing the system of user intent to trigger specific actions. For example, existing mechanisms of user input can be mapped to navigation-specific contexts to facilitate system triggering. As one example, a video-based navigation system configured to implement the principles of the present disclosure may include devices such as an arthroscopic video camera head and console control unit (CCU), a video-based navigation (VBN) controller, a tablet, a mechanical resection controller and handpiece, a coblation controller and handpiece, a fluid management pump, a footswitch to control the mechanical resection, a coblation system, and/or other connected devices and instruments. Software executing on the VBN controller is initiated by the user, and user input is typically required to trigger workflow progression and specific actions (e.g. registration, targeting, etc.). In one example implementation according to the principles of the present disclosure, at all steps of operation of the system, there is a tight coupling between system and/or application state, software activity, and the action being performed by the user. For example, the system state is responsive to and dependent upon user actions (e.g., connecting a specific tool, selecting a specific action, etc.), but the action performed by the system in response to a user input is further dependent upon the system state at the time a specific tool or action is selected by the user (and detected by the system).

Accordingly, in an example, an arthroscopic video-based navigation system according to the present disclosure includes an instrument or tool including camera head buttons that are mapped to different application activities, functions, and states. An example camera head system implementing application-specific mappings according to the principles of the present disclosure is described below in more detail. For example, each button or other input may have one or more states that are fixed or vary in accordance with a state of the application. A button with a fixed state may always have the same type of action (e.g., capture image, capture video, etc.), which may be the same or different for both short and long button presses. Conversely, a button with variable states may have different actions for different application states (e.g., in a first state, a left-short button press is mapped to a "forward" action to advance system or software state while a left-long button press is mapped to a "reverse" action to reverse system or software state, and in a second state the left-short button press and the left-long button press are mapped to different functions dependent upon the second state). Application states may be selected and/or progressed automatically in response to detecting various instruments or tools in a surgical environment.

In another example implementation, the navigation system and a probe device are modified to facilitate integration and detection of user inputs at a handpiece/hub. Example probes according to the present disclosure are modified to enable seamless integration with the surgeon, handpiece, and application interface as describe below in more detail. The probe can be configured to couple to existing handpiece/hub controls. In other words, the same handpiece can be coupled to different device types, such as the probe, a blade or other cutting tool, etc.

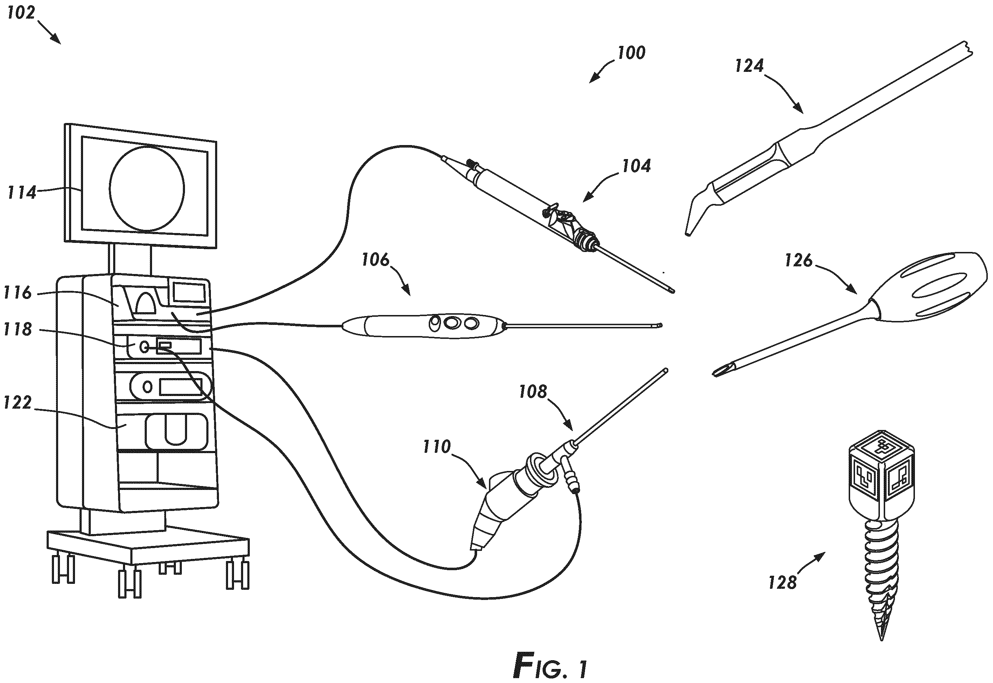

Figure 1 shows an example surgical system (e.g., a system including or implementing an arthroscopic video-based navigation system; not to scale) 100 in accordance with at least some embodiments of the present disclosure. In particular, the example surgical system 100 comprises a tower or device cart 102, an example mechanical resection instrument 104, an example plasma-based ablation instrument (hereafter just ablation instrument 106), and an endoscope in the example form of an arthroscope 108 and attached camera head or camera 110. In the example systems, the arthroscope 108 is a rigid device, unlike endoscopes for other procedures, such as upper-endoscopies. The device cart 102 may comprise a display device 114, a resection controller 116, and a camera control unit (CCU) together with an endoscopic light source and video (e.g., a VBN) controller 118. In example cases the combined CCU and video controller 118 not only provides light to the arthroscope 108 and displays images received from the camera 110, but also implements various additional aspects, such as registering a three-dimensional bone model with the bone visible in the video images, and providing computer-assisted navigation during the surgery. Thus, the combined CCU and video controller are hereafter referred to as surgical controller 118. In other cases, however, the CCU and video controller may be a separate and distinct system from the controller that handles registration and computer-assisted navigation, yet the separate devices would nevertheless be operationally coupled.

The example device cart 102 further includes a pump controller 122 (e.g., single or dual peristaltic pump). Fluidic connections of the mechanical resection instrument 104 and ablation instrument 106 to the pump controller 122 are not shown so as not to unduly complicate the figure. Similarly, fluidic connections between the pump controller 122 and the patient are not shown so as not to unduly complicate the figure. In the example system, both the mechanical resection instrument 104 and the ablation instrument 106 are coupled to the resection controller 116 being a dual-function controller. In other cases, however, there may be a mechanical resection controller separate and distinct from an ablation controller. The example devices and controllers associated with the device cart 102 are merely examples, and other examples include vacuum pumps, patient-positioning systems, robotic arms holding various instruments, ultrasonic cutting devices and related controllers, patient-positioning controllers, and robotic surgical systems.

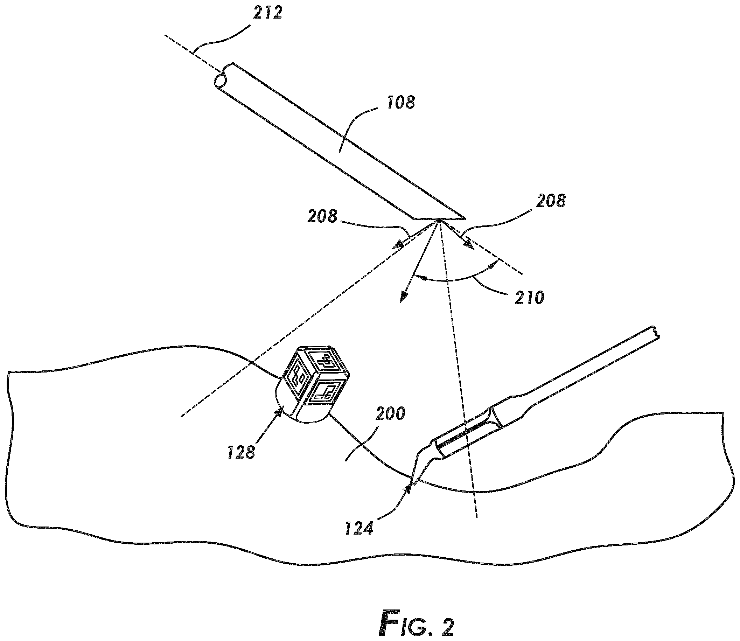

Figures 1 and 2 further show additional instruments that may be present during an arthroscopic surgical procedure. In particular, an example probe 124 (e.g., shown as a touch probe, but which may be a touchless probe in other examples), a drill guide or aimer 126, and a bone fiducial 128 are shown. The probe 124 may be used during the surgical procedure to provide information to the surgical controller 118, such as information to register a three-dimensional bone model to an underlying bone visible in images captured by the arthroscope 108 and camera head 110. The aimer 126 may be used as a guide for placement and drilling with a drill wire to create an initial or pilot tunnel through the bone. The bone fiducial 128 may be affixed or rigidly attached to the bone and serve as an anchor location for the surgical controller 118 to know the orientation of the bone (e.g., after registration of a three-dimensional bone model). Additional tools and instruments will be present, such as the drill wire, various reamers for creating the throughbore and counterbore aspects of a tunnel through the bone, and various tools, such as for suturing and anchoring a graft. These additional tools and instruments are not shown so as not to further complicate the figure. The specification now turns to a workflow for an example anterior cruciate ligament repair.

A surgical procedure may begin with a planning phase. The example anterior cruciate ligament repair may start with imaging (e.g., X-ray imaging, computed tomography (CT), magnetic resonance imaging (MRI)) of the knee of the patient, including the relevant anatomy like the lower portion of the femur, the upper portion of the tibia, and the articular cartilage. The imaging may be preoperative imaging, hours or days before the intraoperative repair, or the imaging may take place within the surgical setting just prior to the intraoperative repair. The discussion that follows assumes MRI imaging, but again many different types of imaging may be used. The image slices from the MRI imaging can be segmented such that a volumetric model or three-dimensional model of the anatomy is created. Any suitable currently available, or after developed, segmentation technology may be used to create the three-dimensional model. More specifically to the example of anterior cruciate ligament repair, a three-dimensional bone model of the lower portion of the femur, including the femoral condyles, is created.

Using the three-dimensional bone model, an operative plan is created that comprises choosing a planned-tunnel path through the femur, including locations of the apertures of the bone that define the ends of the tunnel. In some cases, a three-dimensional bone model of the proximal end of the tibia is also created, and the surgeon may likewise choose planned-tunnel path(s) through the tibia. The results of the planning may include: a three-dimensional bone model of the distal end of the femur; a three-dimensional bone model for a proximal end of the tibia; an entry location and exit location through the femur and thus a planned-tunnel path for the femur; and an entry location and exit location through the tibia and thus a planned-tunnel path through the tibia. Other surgical parameters may also be selected during the planning, such as tunnel throughbore diameters, tunnel counterbore diameters and depth, desired post-repair flexion, and the like, but those additional surgical parameters are omitted so as not to unduly complicate the specification.

The intraoperative aspects include steps and procedures for setting up the surgical system to perform the various repairs. It is noted, however, that some of the intraoperative aspects (e.g., optical system calibration) may take place before any ports or incisions are made through the patient's skin, and in fact before the patient is wheeled into the surgical room. Nevertheless, such steps and procedures may be considered intraoperative as they take place in the surgical setting and with the surgical equipment and instruments used to perform the actual repair.

The example ACL repair is conducted arthroscopically and is computer-assisted in the sense the surgical controller 118 is used for arthroscopic navigation within the surgical site. More particularly, in example systems the surgical controller 118 provides computer-assisted navigation during the ligament repair by tracking location of various objects within the surgical site, such as the location of the bone within the three-dimensional coordinate space of the view of the arthroscope, and location of the various instruments (e.g., a drill wire) within the three-dimensional coordinate space of the view of the arthroscope. A brief description of such tracking techniques is described below.

Figure 2 shows a conceptual drawing of a surgical site with various objects (e.g., surgical instruments/tools) within the surgical site. In particular, visible in Figure 2 is a distal end of the arthroscope 108, a portion of a bone 200 (e.g., femur), the bone fiducial 128 within the surgical site, and the probe 124.

The arthroscope 108 illuminates the surgical site with visible light. In the example of Figure 2, the illumination is illustrated by arrows 208. The illumination provided to the surgical site is reflected by various objects and tissues within the surgical site, and the reflected light that returns to the distal end enters the arthroscope 108, propagates along an optical channel within the arthroscope 108, and is eventually incident upon a capture array within the camera 110 (Figure 1). The images detected by the capture array within the camera 110 are sent electronically to the surgical controller 118 (Figure 1) and displayed on the display device 114 (Figure 1). In one example, the arthroscope 108 is monocular or has a single optical path through the arthroscope for capturing images of the surgical site, notwithstanding that the single optical path may be constructed of two or more optical members (e.g., glass rods, optical fibers). That is to say, in example systems and methods the computer-assisted navigation provided by the arthroscope 108, the camera 110, and the surgical controller 118 is provided with the arthroscope 108 that is not a stereoscopic endoscope having two distinct optical paths separated by an interocular distance at the distal end endoscope.

During a surgical procedure, a surgeon selects an arthroscope with a viewing direction beneficial for the planned surgical procedure. Viewing direction refers to a line residing at the center of an angle subtended by the outside edges or peripheral edges of the view of an endoscope. The viewing direction for some arthroscopes is aligned with the longitudinal central axis of the arthroscope, and such arthroscopes are referred to as "zero degree" arthroscopes (e.g., the angle between the viewing direction and the longitudinal central axis of the arthroscope is zero degrees). The viewing direction of other arthroscopes forms a non-zero angle with the longitudinal central axis of the arthroscope. For example, for a 30° arthroscope the viewing direction forms a 30° angle to the longitudinal central axis of the arthroscope, the angle measured as an obtuse angle beyond the distal end of the arthroscope. In many cases for ACL repair, the surgeon selects a 30° arthroscope or a 45° arthroscope based on location the port created through the skin of the patient. In the example of Figure 2, the view angle 210 of the arthroscope 108 forms a non-zero angle to the longitudinal central axis 212 of the arthroscope 108.

Still referring to Figure 2, within the view of the arthroscope 108 is a portion of the bone 200 (e.g., within the intercondylar notch), along with the example bone fiducial 128, and the example probe 124. The example bone fiducial 128 is multi-faceted element, with each face or facet having a fiducial disposed or created thereon. However, the bone fiducial need not have multiple faces, and in fact may take any shape so long as that shape can be tracked within the video images. The bone fiducial, such as bone fiducial 128, may be attached to the bone 200 in any suitable form, in this example the fastening by the screw portion of the bone fiducial 128 (not visible in Figure 2, but visible in Figure 1). The patterns of the fiducials on each facet are designed to provide information regarding the orientation of the bone fiducial 128 in the three-dimensional coordinate space of the view of the arthroscope 108. More particularly, the pattern is selected such that the orientation of the bone fiducial 128 may be determined from images captured by the arthroscope 108 and attached camera (Figure 1).

The probe 124 is also shown as partially visible within the view of the arthroscope 108. The probe 124 may be used, as discussed more below, to identify a plurality of surface features on the bone 200 as part of the registration of the bone 200 to the three-dimensional bone model. Alternatively, though not specifically shown, the aimer 126 (Figure 1) may be used as the device to help with the registration process. In some cases the probe 124 and/or the aimer 126 may carry their own, unique fiducials, such that their respective poses may be calculated from the one or more fiducial present in the video stream. However, in other cases, and as shown, the medical instrument used to help with registration of the three-dimensional bone model, be it the probe 124, the aimer 126, or any other suitable medical device, may omit carrying fiducials. Stated otherwise, in such examples the medical instrument has no fiducial markings. In such cases, the pose of the medical instrument may be determined by a machine learning model, discussed in more detail below.

The images captured by the arthroscope 108 and attached camera are subject to optical distortion in many forms. For example, the visual field between distal end of the arthroscope 108 and the bone 200 within the surgical site is filled with fluid, such as bodily fluids and saline used to distend the joint. Many arthroscopes have one or more lenses at the distal end that widen the field of view, and the wider field of view causes a "fish eye" effect in the captured images. Further, the optical elements within the arthroscope (e.g., rod lenses) may have optical aberrations inherent to the manufacturing and/or assembly process. Further still, the camera may have various optical elements for focusing the images received onto the capture array, and the various optical elements may have aberrations inherent to the manufacturing and/or assembly process. In example systems, prior to use within each surgical procedure, the endoscopic optical system is calibrated to account for the various optical distortions. The calibration creates a characterization function that characterizes the optical distortion, and further analysis of the frames of the video stream may be, prior to further analysis, compensated using the characterization function.

The next example step in the intraoperative procedure is the registration of the bone model created during the planning stage. During the intraoperative repair, the three-dimensional bone model is obtained by or provided to the surgical controller 118. Again using the example of anterior cruciate ligament repair, and specifically computer-assisted navigation for tunnel paths through the femur, the three-dimensional bone model of the lower portion of the femur is obtained by or provided to the surgical controller 118. Thus, the surgical controller 118 receives the three-dimensional bone model, and assuming the arthroscope 108 is inserted into the knee by way of a port through the patient's skin, the surgical controller 118 also receives video images of a portion of the lower end of the femur. In order to relate the three-dimensional bone model to the images received by way of the arthroscope 108 and camera 110, the surgical controller 118 registers the three-dimensional bone model to the images of the femur received by way of the arthroscope 108 and camera 110.

In order to perform the registration, and in accordance with example methods, the bone fiducial 128 is attached to the femur. The bone fiducial placement is such that the bone fiducial is within the field of view of the arthroscope 108, but in a location spaced apart from the expected tunnel entry/exit point through the lateral condyle. More particularly, in example cases the bone fiducial 128 is placed within the intercondylar notch superior to the expected location of the tunnel through lateral condyle. To relate or register bone visible in the video images to the three-dimensional bone model, the surgical controller 118 (Figure 1) is provided or determines a plurality of surface features of an outer surface of the bone. Identifying the surface features may take several forms, including a touch-based registration using the probe 124 without a carried fiducial, a touchless registration technique in which the surface features are identified after resolving the motion of the arthroscope 108 and camera relative to the bone fiducial 128, and a third technique in which uses a patient-specific instrument.

In the example touch-based registration, the surgeon may touch a plurality of locations using the probe 124 (Figure 1). In some cases, particularly when portions of the outer surface of the bone are exposed to view, receiving the plurality of surface features of the outer surface of the bone may involve the surgeon "painting" the outer surface of the bone. "Painting" is a term of art that does not involve application of color or pigment, but instead implies motion of the probe 124 when the distal end of the probe 124 is touching bone. In this example, the probe 124 does not carry or have a fiducial visible to the arthroscope 108 and the camera 110. It follows that the pose of the probe 124 and the location of the distal tip of the probe 124 needs to be determined in order to gather the surface features for purposes of registering the three-dimensional bone model.

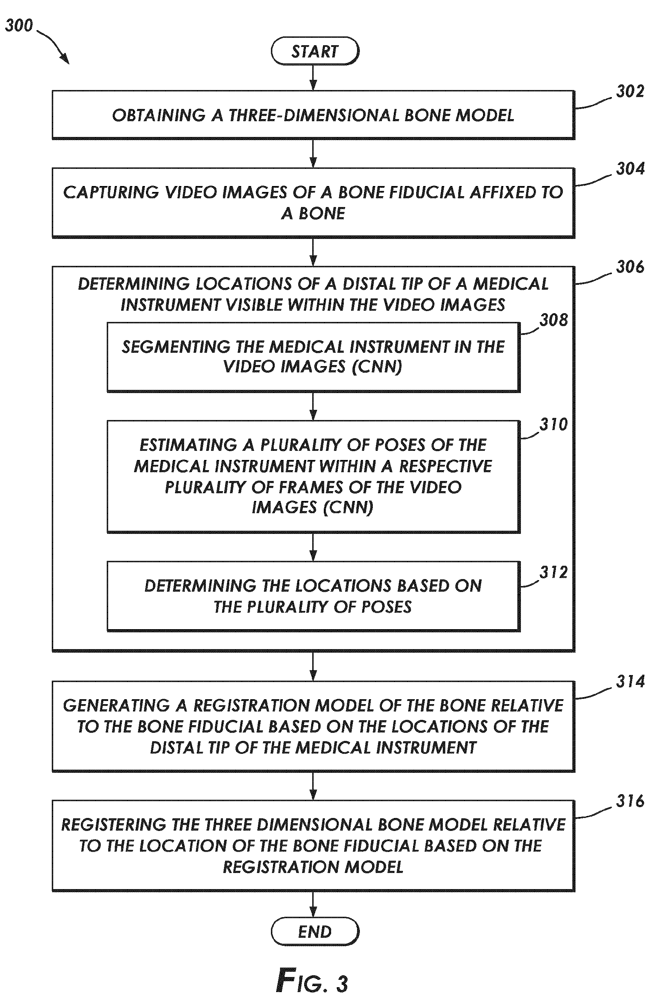

Figure 3 shows a method 300 in accordance with at least some embodiments of the present disclosure. The example method 300 may be implemented in software within a computer system, such as the surgical controller 118. In particular, the example method 300 comprises obtaining a three-dimensional bone model (block 302). That is to say, in the example method 300, what is obtained is the three-dimensional bone model that may be created by segmenting a plurality of non-invasive images (e.g., CT, MRI) taken preoperatively or intraoperatively. With the bone segmented from or within the images, the three-dimensional bone model may be created. The three-dimensional bone may take any suitable form, such as a computer-aided design (CAD) model, a point cloud of data points with respect to an arbitrary origin, or a parametric representation of a surface expressed using analytical mathematical equations. Thus, the three-dimensional bone model is defined with respect to the origin and in any suitable an orthogonal basis.

The next step in the example method 300 is capturing video images of the bone fiducial attached to the bone (block 304). The capturing is performed intraoperatively. In the example case of an arthroscopic anterior cruciate ligament repair, the capturing of video images is by way of the arthroscope 108 and camera 110. Other endoscopes may be used, such as endoscopes in which the capture array resides at the distal end of the device (e.g., chip-on-the-tip devices). However, in open procedures where the skin is cut and pulled away, exposing the bone to the open air, the capturing may be by any suitable camera device, such as one or both cameras of a stereoscopic camera system, or a portable computing device, such as a tablet or smart-phone device. The video images may be provided to the surgical controller 118 in any suitable form.

The next step in the example method 300 is determining locations of a distal tip of the medical instrument visible within the video images (block 306), where the distal tip is touching the bone in at least some of the frames of the video images, and the medical instrument does not have a fiducial. Determining the locations of the distal tip of the medical instrument may take any suitable form. In one example, determining the locations may include segmenting the medical instrument in the frames of the video images (block 308). The segmenting may take any suitable form, such as applying the video images to a segmentation machine learning algorithm. The segmentation machine learning algorithm may take any suitable form, such as neural network or convolution neural network trained with a training data set showing the medical instrument in a plurality of known orientations. The segmentation machine learning algorithm may produce segmented video images where the medical instrument is identified or highlighted in some way (e.g., box, brightness increased, other objects removed).

With the segmented video images, the example method 300 may estimate a plurality of poses of the medical instrument within a respective plurality of frames of the video images (block 310). The estimating the poses may take any suitable form, such as applying the video images to a pose machine learning algorithm. The pose machine learning algorithm may take any suitable form, such as neural network or convolution neural network trained to perform six-dimensional pose estimation. The resultant of the pose machine learning algorithm may be, for at least some of the frames of the video image, an estimated pose of the medical instrument in the reference frame of the video images and/or in the reference frame provided by the bone fiducial. That is, the resultant of the pose machine learning algorithm may be a plurality of poses, one pose each for at least some of the frames of the segmented video images. While in many cases a pose may be determined for each frame, in other cases it may not be possible to make a pose estimation for at least some frame because of video quality issues, such as motion blur caused by electronic shutter operation.

The next step in the example method 300 is determining the locations based on the plurality of poses (block 312). In particular, for each frame for which a pose can be estimated, based on a model of the medical device the location of the distal tip can be determined in the reference frame of the video images and/or the bone fiducial. Thus, the resultant is a set of locations that, at least some of which, represent locations of the outer surface of the bone.

The Figure 3 shows an example three-step process for determining the locations of the distal tip of the medial instrument. However, the method 300 is merely an example, and many variations are possible. For example, a single machine learning model, such as a convolution neural network, may be set up and trained to perform all three steps as a single overall process, though there may be many hidden layers of the convolution neural network. That is, the convolution neural network may segment the medical instrument, perform the six-dimensional pose estimation, and determine the location of the distal tip in each frame. The training data set in such a situation would include a data set in which each frame has the medical device segmented, the six-dimensional pose identified, and the location of the distal tip identified. The output of the determining step 306 may be a segmented video stream distinct from the video images captured at step 304. In such cases, the later method steps may use both segmented video stream and the video images to perform the further tasks. In other cases, the location information may be combined with the video images, such as being embedded in the video images, or added as metadata to each frame of the video images.

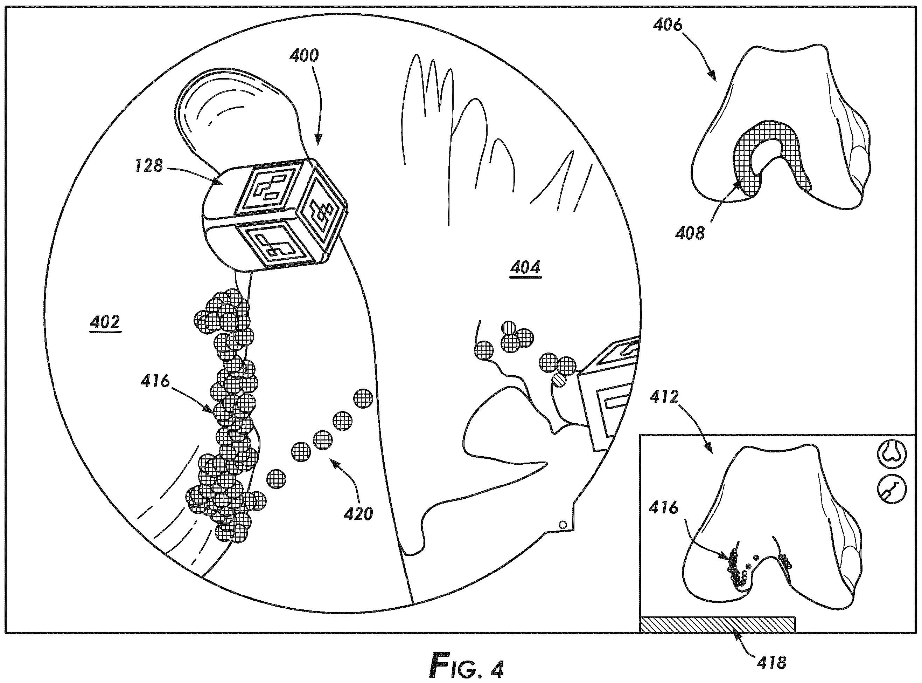

Figure 4 is an example video display showing portions of a femur and a bone fiducial during a registration procedure. The display may be shown, for example, on the display device 114 associated with the device cart 102, or any other suitable location. In particular, visible in the main part of the display of Figure 4 is an intercondylar notch 400, a portion of the lateral condyle 402, a portion the medial condyle 404, and the example bone fiducial 128. Shown in the upper right corner of the example display is a depiction of the bone, which may be a rendering 406 of the bone created from the three-dimensional bone model. Shown on the rendering 406 is a recommended area 408, the recommended area 408 being portions of the surface of the bone to be "painted" as part of the registration process. Shown in the lower right corner of the example display is a depiction of the bone, which again may be a rendering 412 of the bone created from the three-dimensional bone model. Shown on the rendering 412 are a plurality of surface features 416 on the bone model that have been identified as part of the registration process. Further shown in the lower right corner of the example display is progress indicator 418, showing the progress of providing and receiving of locations on the bone. The example progress indicator 418 is a horizontal bar having a length that is proportional to the number of locations received, but any suitable graphic or numerical display showing progress may be used (e.g., 0% to 100%).

Referring to both the main display and the lower right rendering, as the surgeon touches the outer surface of the bone within the images captured by the arthroscope 108 and camera 110, the surgical controller 118 receives the surface features on the bone, and may display each location both within the main display as dots or locations 416, and within the rendering shown in the lower right corner. More specifically, the example surgical controller 118 overlays indications of identified surface features 416 on the display of the images captured by the arthroscope 108 and camera 110, and in the example case shown, also overlays indications of identified surface features 416 on the rendering 412 of the bone model. Moreover, as the number of identified locations 416 increases, the surgical controller 118 also updates the progress indicator 418.

Still referring to Figure 4, in spite of the diligence of the surgeon, not all locations identified by the surgical controller 118 based on the surgeon's movement of the probe 124 result in valid locations on the surface of the bone. In the example of Figure 4, as the surgeon moves the probe 124 from the inside surface of the lateral condyle 102 to the inside surface of the medial condyle 104, the surgical controller 118, based on the example six-dimensional pose estimation, receives several locations 420 that likely represent locations at which the distal end of the probe 124 was not in contact with the bone.

With reference to Figure 3, the plurality of surface features 416 may be, or the example surgical controller 118 may generate, a registration model relative to the bone fiducial 128 (block 314). The registration model may take any suitable form, such as a computer-aided design (CAD) model or point cloud of data points in any suitable orthogonal basis. The registration model, regardless of the form, may have fewer overall data points or less "structure" than the bone model created by the non-invasive computer imaging (e.g., MRI). However, the goal of the registration model is to provide the basis for the coordinate transforms and scaling used to correlate the bone model to the registration model and relative to the bone fiducial 128. Thus, the next step in the example method 300 is registering the bone model relative to the location of the bone fiducial based on the registration model (block 316). Registration may conceptually involve testing a plurality of coordinate transformations and scaling values to find a correlation that has a sufficiently high correlation or confidence factor. Once a correlation is found with the sufficiently high confidence factor, the bone model is said to be registered to the location of the bone fiducial. Thereafter, the example registration method 300 may end (block 318); however, the surgical controller 118 may then use the registered bone model to provide computer-assisted navigation regarding a procedure involving the bone.

In the examples discussed to this point, registration of the bone model involves a touch-based registration technique using the probe 124 without a carried fiducial. However, other registration techniques are possible, such as a touchless registration technique. The example touchless registration technique again relies on placement of the bone fiducial 128. As before, when the viewing direction of the arthroscope 108 is relatively constant, the bone fiducial may have fewer faces with respective fiducials. Once placed, the bone fiducial 128 represents a fixed location on the outer surface of the bone in the view of the arthroscope 108, even as the position of the arthroscope 108 is moved and changed relative to the bone fiducial 128. Again, in order to relate or register the bone visible in the video images to the three-dimensional bone model, the surgical controller 118 (Figure 1) determines a plurality of surface features of an outer surface of the bone, and in this example determining the plurality of surface features is based on a touchless registration technique in which the surface features are identified based on motion of the arthroscope 108 and camera 110 relative to the bone fiducial 128.

Another technique for registering the bone model to the bone uses a patient-specific instrument. In both touch-based and touchless registration techniques, a registration model is created, and the registration model is used to register the bone model to the bone visible in the video images. Conceptually, the registration model is used to determine a coordinate transformation and scaling to align the bone model to the actual bone. However, if the orientation of the bone in the video images is known or can be determined, use of the registration model may be omitted, and instead the coordinate transformations and scaling may be calculated directly.

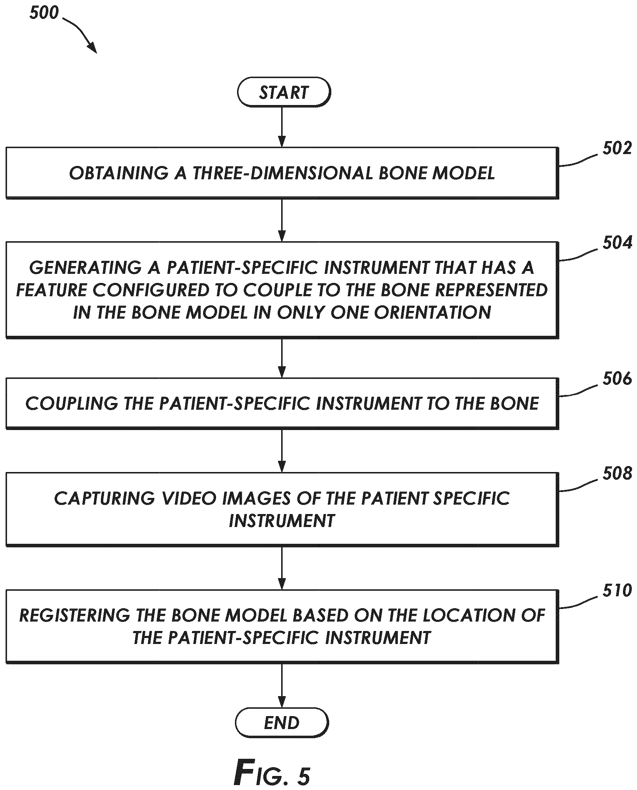

Figure 5 shows a method 500 in accordance with at least some embodiments. The example method may be implemented in software within one or more computer systems, such as, in part, the surgical controller 118. In particular, the example method 500 starts and comprises obtaining a three-dimensional bone model (block 502). Much like the prior techniques, in the patient-specific instrument registration technique what is obtained is the three-dimensional bone model that may be created by segmenting a plurality of non-invasive images (e.g., MRI) taken preoperatively or intraoperatively.

The next step in the example method 500 is generating a patient-specific instrument that has a feature designed to couple to the bone represented in the bone model in only one orientation (block 504). Generating the patient-specific instrument may first involve selecting a location at which the patient-specific instrument will attach. For example, a device or computer system may analyze the bone model and select the attachment location. In various examples, the attachment location may be a unique location in the sense that, if a patient-specific instrument is made to couple to the unique location, the patient-specific instrument will not couple to the bone at any other location. In the example case of an anterior cruciate ligament repair, the location selected may be at or near the upper or superior portion on the intercondylar notch. If the bone model shows another location with a unique feature, such as a bone spur or other raised or sunken surface anomaly, such a unique location may be selected as the attachment location for the patient-specific instrument.

Moreover, forming the patient-specific instrument may take any suitable form. In one example, a device or computer system may directly print, such as using a 3D printer, the patient-specific instrument. In other cases, the device or computer system may print a model of the attachment location, and the model may then become the mold for creating the patient-specific instrument. For example, the model may be the mold for an injection-molded plastic or casting technique. In some examples, the patient-specific instrument carries one or more fiducials, but as mentioned above, in other cases the patient-specific instrument may itself be tracked and thus carry no fiducials.

The next step in the example method 500 is coupling the patient-specific instrument to the bone, in some cases the patient-specific instrument having the fiducial coupled to an exterior surface (block 506). As previously mentioned, the attachment location for the patient-specific instrument is selected to be unique such that the patient-specific instrument couples to the bone in only one location and in only one orientation. In the example case of an arthroscopic ACL repair, the patient-specific instrument may be inserted arthroscopically. That is, the attachment location may be selected such that a physical size of the patient-specific instrument enables insertion through the ports in the patient's skin. In other case, the patient-specific instrument may be made or constructed of a flexible material that enables the patient-specific instrument to deform for insertion in the surgical site, yet return to the predetermined shape for coupling to the attachment location. However, in open procedures where the skin is cut and pulled away, exposing the bone to the open air, the patient-specific instrument may be a rigid device with fewer size restrictions.

The next step in the example method 500 is capturing video images of the patient-specific instrument (block 508). Here again, the capturing may be performed intraoperatively. In the example case of an arthroscopic anterior cruciate ligament repair, the capturing of video images is by the surgical controller 118 by way of arthroscope 108 and camera 110. However, in open procedures where the skin is cut and pulled away, exposing the bone to the open air, the capturing may be by any suitable camera device, such as one or both cameras of a stereoscopic camera systems, or a portable computing device, such as a tablet or smart-phone device. In such cases, the video images may be provided to the surgical controller 118 in any suitable form.

The next step in the example method 500 is registering the bone model based on the location of the patient-specific instrument (block 510). That is, given that the patient-specific instrument couples to the bone at only one location and in only one orientation, the location and orientation of the patient-specific instrument is directly related to the location and origination of the bone, and thus the coordinate transformations and scaling for the registration may be calculated directly. Thereafter, the example method 500 may end; however, the surgical controller 118 may then use the registered bone model to provide computer-assisted navigation regarding a surgical task or surgical procedure involving the bone.

For example, with the registered bone model the surgical controller 118 may provide guidance regarding a surgical task of a surgical procedure. The specific guidance is dependent upon the surgical procedure being performed and the stage of the surgical procedure. A non-exhaustive list of guidance comprises: changing a drill path entry point; changing a drill path exit point; aligning an aimer along a planned drill path; showing location at which to cut and/or resect the bone; reaming the bone by a certain depth along a certain direction; placing a device (suture, anchor or other) at a certain location; placing a suture at a certain location; placing an anchor at a certain location; showing regions of the bone to touch and/or avoid; and identifying regions and/or landmarks of the anatomy. In yet still other cases, the guidance may include highlighting within a version of the video images displayed on a display device, which can be the arthroscopic display or a see-through display, or by communicating to a virtual reality device or a robotic tool.

An example of the surgical system 100 and corresponding methods according to the principles of the present disclosure are configured to facilitate functions related to informing the system of user intent to trigger specific actions using various surgical instruments. As presented below, the principles of the present disclosure are described with respect to configurations and functions of the arthroscope 108 and camera/camera head 110. However, the principles of the present disclosure may also be applied to other types of surgical instruments, such as the instruments 104, 106, 124, 126, etc. For example, mechanisms of user input of the camera 110 are mapped to navigation-specific contexts to facilitate triggering of various system functions. Software (e.g., an application) executing on a controller of the system 100 (e.g., on a VBN controller) is initiated by the user prior to or during various preoperative or intraoperative procedures involving one or more of the surgical instruments. During the procedure and subsequent to activation/initiation of the software, additional user input may be required to trigger workflow progression and specific actions (e.g. registration, targeting, image capture, etc.). Accordingly, functions of the system 100 associated with respective instruments are dynamically mapped to input mechanisms of the instruments based on system and software/application states, software activity, and actions being performed by the user. For example, the system and software states are responsive to and dependent upon user actions (e.g., connecting a specific tool, selecting a specific action, etc.), but the action performed by the system is further dependent upon the system state at the time a specific tool or action is selected by the user (i.e., rather than simply being fixed for each input mechanism and instrument). As one example, system and software states may be response to, at least in part, detection of various surgical instruments in a surgical environment. For example, presence or absence of various instruments may be indicative of the intent of the surgeon, next steps to be performed by the surgeon, desired functionality of an instrument being used by the surgeon, etc. Dynamic mapping of input mechanisms as described herein may be referred to as "application-specific mapping."

An example camera, camera head, and/or camera system implementing application-specific mappings according to the principles of the present disclosure, such as the camera or camera head 110, are described below in more detail.

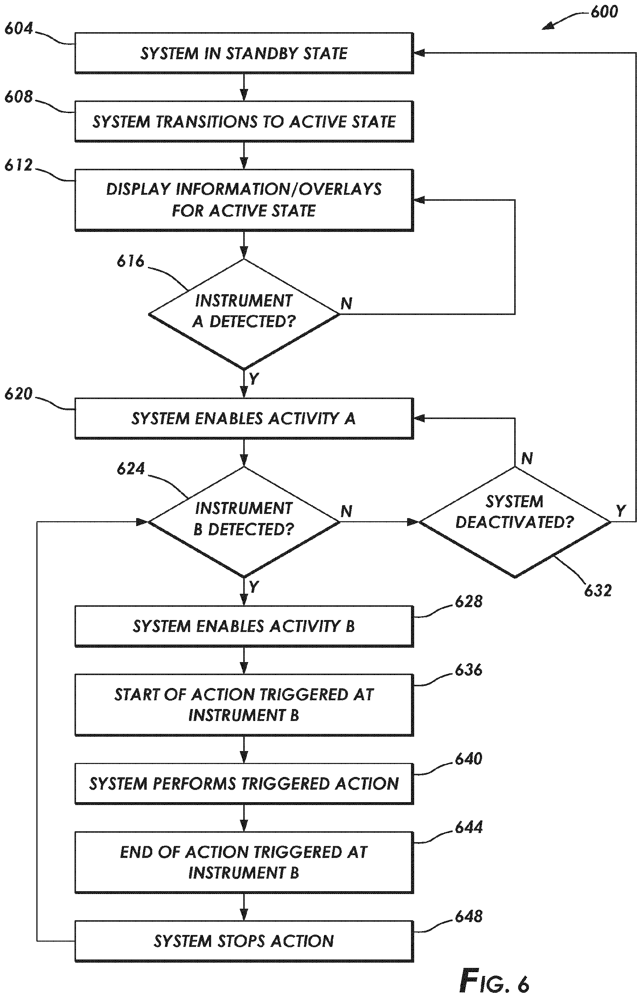

Figure 6 shows an example method 600 for progressing between various system states (e.g., operational states of the system 100) and enabling various activities, which may enable and/or be responsive to user actions (e.g., actions performed using the system 100 and/or one or more of the surgical instruments coupled to the system 100, such as the camera head 110). As used herein, "instrument" may refer to tools or instruments having one or more input mechanisms (e.g., a camera head with one or more buttons), tools or instruments without active input mechanisms (e.g. aligners, aimers, etc.), and/or "passive" or stationary tools such as fiducials, markers, etc. that may be arranged in fixed locations within the surgical environment.

In this example, the method 600 will be described in the context of a procedure involving multiple tools or instruments, such as performed by the systems and methods described above in Figures 1-5. For example, in the method 600, the instrument A may correspond to a bone fiducial (e.g., the bone fiducial 128) and a camera head or camera (e.g., the camera 110). Further, although described with respect to two instruments (A and B), the method 600 may be performed using only one instrument, more than two instruments, etc.

At 604, the system (e.g., a surgical system 100, implementing a software application associated with a surgical procedure) is in a standby state. For example, the system is in the standby state prior to a user turning on the system, an application, etc. In response to the user turning on the system and/or application, the system transitions to an active state at 608.

In the active state, the system is responsive to user commands to perform functions using one or more instruments, change application states, etc. More specifically, in the active state the system may be responsive a subset of commands to perform some actions, dependent upon a selected application state. For example, in a first application state, the system may be configured to be responsive to a first subset of commands and, in a second application state, the system may be configured to be responsive to a second subset of commands. Further, depending on the application state, inputs/commands provided by the user via an instrument may differ (e.g., a same buttons may correspond to different commands/functions in different application states).

At 612, the system may display information and/or overlays corresponding to the active state. For example, the displayed information may indicate a current application state, functions available to various instruments in the current application state, commands/functions assigned to various inputs/buttons of instruments in the current application state, etc. Although only shown in step 612, the display of information may be performed throughout the method 600 such that the display may change as various states, activities, detected instruments, etc. are changed.

At 616, the method 600 determines whether the instrument A is detected. As used herein, detecting an instrument may correspond to detecting an instrument in a surgical scene or environment using a camera, such as detecting a fiducial or other marker on an instrument in the surgical environment, using image analysis to detect and identify objects in a captured image of the surgical environment, detecting use and/or introduction of an instrument (e.g., by a surgeon) into the surgical environment, etc. In this example, detecting the instrument A may correspond to detecting the fiducial marker, which may indicate that the user is preparing to perform one or more functions related to an arthroscopic procedure, such as registration, image or video capture, etc., which may include the use of one or more additional instruments. In response to detecting the instrument A, the method 600 proceeds to 620. Otherwise, the method 600 may proceed to 612. For example, the method 600 may repeat steps 612 and 616 until the instrument A is detected.

At 620, the method 620 enables a first activity (e.g., activity A). Enabling the activity A may correspond the system transitioning the application to a state (a first state or configuration) in which the activity A is enabled. In the context of the instrument A corresponding to a fiducial marker, activity A may include tracking the fiducial marker as described herein. Once enabled, activity A may be enabled throughout remaining steps of the method 600.

At 624, the method 600 determines whether an instrument B is detected. In this example, detecting the instrument B may correspond to detecting an aimer or aligner, such as the aimer 126, which may indicate that the user is preparing to perform one or more functions associated with using an aimer during registration. For example, detecting the aimer may correspond to detecting the presence of the aimer in the surgical environment by using image analysis, detecting a fiducial or marker on the aimer, etc. While the instrument B is described in this example as an aimer, the instrument B can be any instrument, tool, system, etc. different from the instrument A and/or different from the surgical system itself. For example, the instrument B may correspond to a probe, grasper, pincher, scissor, punch, microfracture pick, knife, meniscectomy tool, curette, anchor deployment device, biologic deployment device, suture anchor deployment device, arthroscopic blade, arthroscopic RF wand, an arthroscopic coblation wand, a bone fiducial, a fiducial (or set of fiducials) on a specific tool, etc.

In response to detecting the instrument B, the method 600 proceeds to 628. Otherwise, the method 600 may proceed to 632. At 632, the method 600 may determine whether the system has been deactivated (e.g., the system has been powered down or otherwise deactivated by the user, the procedure is complete, etc.). If true, the method 600 may return to the standby state at 604. If false, the method 600 may continue to 620 to await detection of additional instruments, user actions, etc.

At 628, the method 600 enables a second activity (e.g., activity B). Enabling the activity B may correspond the system transitioning the application to a second state or configuration in which the activity B is enabled. While one or more aspects of activity A may still be enabled, enabling activity B in accordance with the principles of the present disclosure may include automatically changing functions associated with one or more inputs or buttons of an instrument.

As one example, prior to detecting instrument B (and, correspondingly, enabling activity B and transitioning the application to the second state), one or more buttons of respective instruments may be assigned/mapped to first respective functions or actions. Conversely, in response to detecting instrument B, enabling activity B, and transitioning the application to the second state, the one or more buttons may be assigned to second respective functions or actions. More simply, a button on an instrument may be automatically assigned different functions/actions based on application state, which in turn may be automatically selected/cycled based on detection of specific instruments in the surgical environment. Although detection of instruments is provided as one example of how the systems and method of the present disclosure automatically advance (and/or reverse) application states, other techniques may be used (e.g., voice activation, other visual or audio cues, user input (e.g., a foot switch), etc. Accordingly, a particular button (e.g., a button on a camera head) may be assigned a first action or function in steps 604-624) but may be assigned a second action or function in steps 628-644. As described herein, assigning different functions to a button may include automatically reprogramming the function assigned to the button within the system, system software, etc. For example, the system may be configured/programmed to perform a particular action in response to an indication, received from the instrument, that the user pressed the button, and the programmed action is automatically changed by the system as the application state changes.

At 636, the method 600 includes receiving an indication that an action was triggered at the instrument B (e.g., by the user pressing a button on the instrument B). At 640, the method 600 includes starting (e.g., using the system) the action triggered at the instrument B. Starting the action includes starting the action assigned to the button in accordance with the current application state. At 644, the method 600 includes receiving (e.g., at the system) an indication that the end of the action was triggered at the instrument B (e.g., by the user releasing the button, pressing the button a second time, pressing the button for a different length of time, etc.). At 648, the method 600 includes stopping (e.g., using the system) the action.

The method 600 may continue to advance through (and/or reverse) application states during the procedure as different tools are used and detected, connected to the system, etc. Although only shown with respect to instruments A and B, the method 600 may include detecting additional instruments (e.g., instruments, C, D, etc.), combinations of instruments, etc. and enabling/selecting associated application states and activities. In the various application states, one or more buttons on respective instruments may be assigned different actions/functions (i.e., may cause different actions to be performed), while other buttons may have fixed or static actions/states. Further, detection of different tools may cause the different system/application states to be selected. In some examples, completing actions with various instruments may automatically cause system/application states to advance.

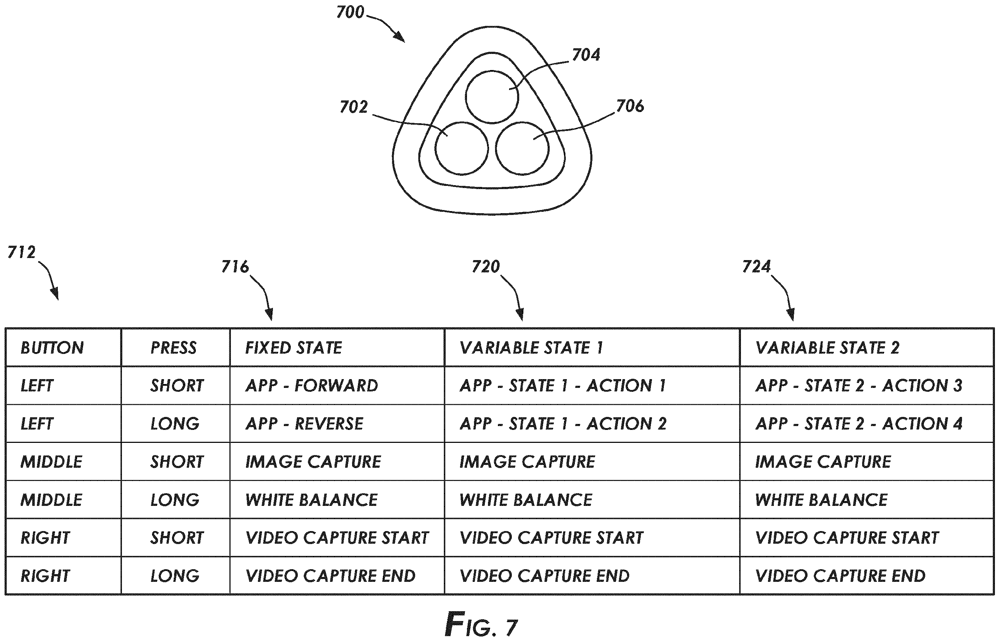

Figure 7 shows an example instrument (e.g., a camera head 700) having a plurality of buttons, such as buttons 702 (e.g., a left button), 704 (e.g., a middle button), and 706 (e.g., a right button), configured to have fixed or variable states in accordance with different application states as described herein. Although described with respect to buttons, the principles of the present disclosure may be implemented with various types of input mechanisms or devices, such as buttons, switches, sliders, a touchpad or other touch-sensitive interface, dials, knobs, etc. As used herein, a "state" of a button may refer to an action or function that is performed (e.g., by the system) in response to the button being pressed. Accordingly, if a button has a fixed state, the corresponding action may remain fixed (i.e., unchanged) regardless of which state the application is in. Conversely, if a button has a variable state, the corresponding action may vary depending on the application state. Further, each button may be assigned different actions in a given state depending upon the manner of actuation of the button. For example, each button may be configured to trigger a first action in response to a short press (e.g., a tap, such as a press less than 0.25 seconds) and to trigger a second action in response to a long press (e.g., a press greater than or equal 0.25 seconds).

A state table 712 illustrates the different actions that may be assigned to the respective buttons and types of button presses (e.g., short and long) in different application states. For reference, an example configuration where each of the buttons has a fixed state regardless of application state is shown at 716. For example, the left button 702 is assigned actions corresponding to moving/advancing the application state forward in response to a short button press and moving/reversing the application state backward in response to a long button press. The middle button 704 is assigned actions corresponding to performing an image capture in response to a short button press and adjusting white balance in response to a long button press. The right button 706 is assigned actions corresponding to initiating/starting video capture in response to a short button press and ending/stopping video capture in response to a long button press.

Conversely, example first and second variable states are shown at 720 and 724. In this example, in the variable states 720, 724, actions assigned to the left button 702 vary in accordance with application state while actions assigned to the middle button 704 and the right button 706 remain fixed. However, in other examples, two or more buttons on a given instrument may have variable states.

In this example, in the first variable state 720 (e.g., corresponding to a first application state), a short press of the left button 702 is assigned a first action ("Action 1") and a long press of the left button 702 is assigned a second action ("Action 2"). Conversely, in the second variable state 724 (e.g., corresponding to a second application state), a short press of the left button 702 is assigned a third action ("Action 3") and a long press of the left button 702 is assigned a fourth action ("Action 4").

In this manner, actions assigned/mapped to one or more buttons of an instrument, such as the buttons 702, 704, 706 of the camera head 700, may vary in accordance with application state without any input or remapping performed by the user, and system or application states may change automatically in response to system detection of various instruments in the surgical environment.

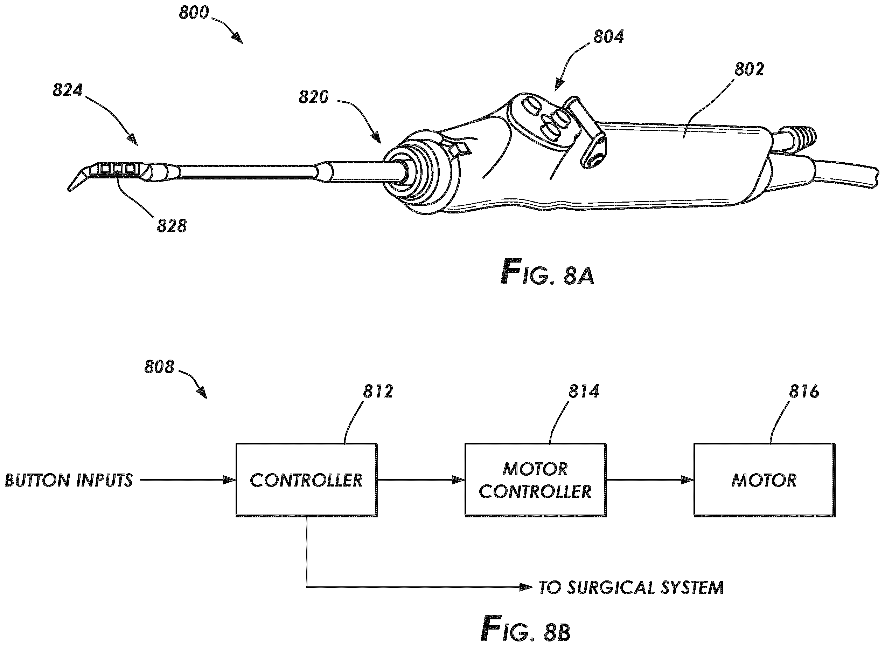

Figures 8A and 8B show another example implementation of an instrument 800 configured for use with a system according to the principles of the present disclosure, such as the surgical system 100. The system and the instrument 800 (shown as a probe device) according to the principles of the present disclosure are modified to facilitate integration and detection of user inputs at a handpiece/hub 802 of the instrument 800. For example, the instrument 800 is modified to enable seamless integration with the surgeon, handpiece, and application interface as describe below in more detail. The instrument 800 can be configured to couple to existing handpiece/hub controls (e.g., existing buttons 804 or other inputs on the handpiece 802. In other words, the same handpiece can be coupled to different device types, such as the probe, a blade or other cutting tool, etc.

Figure 8B shows a functional block diagram of a control system 808 of the instrument 800 (e.g., contained within the handpiece 802). For example, the control system 808 includes a controller 812, a motor controller 814, and a motor 816. The motor controller 814 and the motor 816 may be associated with control of a first device type, such as a shaver, a blade or other cutting tool, a coblation tool, etc. (not shown in Figure 8A or 8B) configured to be mechanically actuated using the instrument 800. With the first device type connected to the handpiece 802 (e.g., plugged/inserted into a connection interface, such as a socket/opening 820 at a distal end of the handpiece 802), pressing the buttons 804 causes various inputs or input signals to be provided to the controller 812 to control and/or be responsive to the first device type. For example, the controller 812 provides various control signals to the motor controller 814 in response to the button inputs, which in turn controls the motor 816 to drive a blade, perform forward or reverse rotation, perform oscillation functions, etc.

Conversely, with a second device type connected to the handpiece 802 (e.g., plugged into the socket 820), pressing the buttons 804 causes various inputs or input signals to be provided to the controller 812 to control and/or be responsive to the second device type. For example, the second device type may correspond to a probe 824 as described herein. Accordingly, in response to button presses with the probed 824 connected to the handpiece 802, the controller 812 provides various control signals to the surgical system, a navigation system, etc. (i.e., instead of controlling the motor controller 814 and the motor 816). For example, functions controllable using the buttons 804 when the probe 824 is connected may include forward progress, reverse progress, capture image, etc. In other words, instead of controlling a device type using internal mechanical components of the instrument 800, with a device type such as the probe 824 connected the controller 812 instead provides signals corresponding to the button inputs to the surgical system to perform various actions/functions associated with the probe 824.

As one example, the controller 812 is configured to detect a type of device connected/coupled to the handpiece 802 via the socket 802 and automatically assign actions/functions to inputs associated with the buttons 804 based on the detected device type. For example, in response to detecting a device type configured to be controlled by the motor controller 814 and the motor 816, the controller 812 is configured to provide control signals to the motor controller 814 in response to the button inputs. Conversely, in response to detecting a device type configured to control application states or actions/functions to be performed by the surgical system/navigation system, the controller 812 is configured to provide control signals to the surgical system (e.g., to a computing device external to the instrument 800). In some examples, actions may be assigned to the buttons 804 by the controller 812. In other examples, actions may be assigned to the buttons 804 by the surgical system. For example, different actions may be assigned to the buttons 804 as described above with respect to Figures 6 and 7. In still other examples, actions may be assigned to the buttons 804 using a user interface of a computing device, such as a tablet.

The type of the device connected to the handpiece 802 may be detected by various techniques, such as detection using a camera/imaging system configured to detect fiducials or other markings 828 on the device, RFID techniques, keying or other mechanical features on the device and/or within the socket 820, electrical or digital communication between the controller 812 and the device, etc. For example, connecting some types of devices to the handpiece 802 via the socket 820 may include only establishing a mechanical coupling between the device and the socket 820. Conversely, connecting other types of devices, such as the probe 824, to the handpiece 802 may further include establishing electrical and/or data connections between the device and the controller 812 via the socket 820.

Although described above with respect to a shaver or other type of cutting device, coblation tool, probe, etc., the principles of the present disclosure may be implemented with other types of devices, such as a tracked micro-fracture tip, a tracked suture anchor punch, a tracked burr, etc. In various examples, some types of devices connected to the handpiece 802 may have full functionality (e.g., all actions associated with controlling the device are assigned to corresponding buttons) or limited functionality (e.g., only some actions associated with controlling the device are assigned to corresponding buttons). For example, for a tracked burr, respective buttons may be assigned forward and reverse actions for resection while another button is assigned actions related to application interface control (e.g., forwarding and reversing application states), omitting control of an oscillation function associated with the tracked burr.

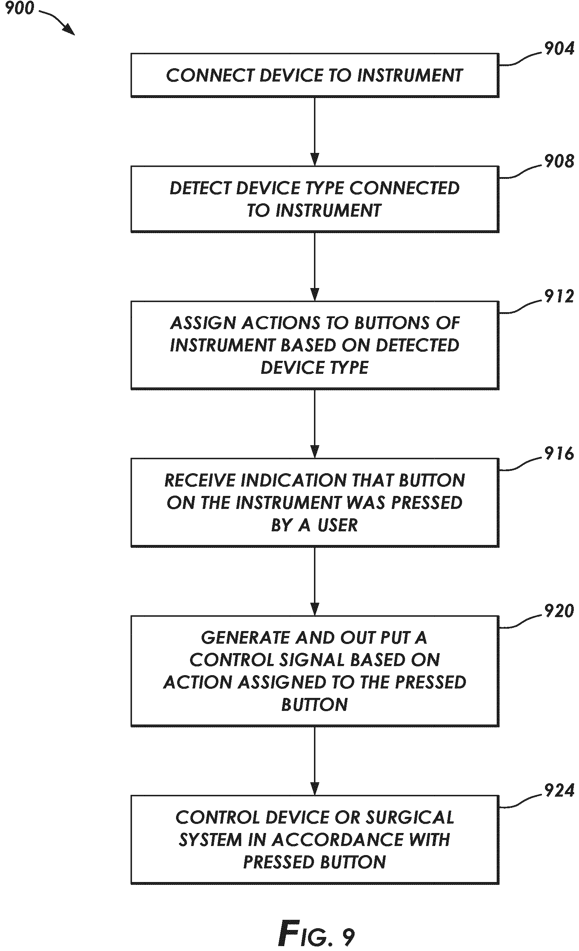

Figure 9 shows an example method 900 for controlling an instrument configured to connect to different device/tool types, such as the instrument 800 of Figures 8A and 8B. At 904, the method 904 includes connecting a tool or device to a handpiece of a surgical instrument. For example, connecting the device to the handpiece includes mechanically coupling the device to a socket (e.g., plugging an end of the device into a socket on a distal end of the handpiece).

At 908, the method 900 includes detecting a device type of the device connected to the handpiece. For example, detecting the device type includes detecting the device type using a controller or other circuitry of the instrument, a surgical system coupled to the instrument, etc. Detecting the device type may include, but is not limited to, detecting the device type based on a mechanical configuration of the device or mechanical engagement between the device and the handpiece (e.g., keying), based on data or other digital communication between the device and the instrument, object detection using a camera or other imaging system, etc.

At 912, the method 900 includes assigning actions to buttons of the instrument based on the detected device type. For example, assigning actions to the buttons may include, but is not limited to, assigning the actions using a controller of the instrument, assigning the actions using a computing device external to the instrument (e.g., a surgical system), etc. Various actions may be assigned in accordance with data (e.g., data stored within memory or other circuitry of the instrument, data stored by the surgical system, etc.) that correlates various device types with corresponding actions, such as a lookup table that correlates detected device types with available actions and corresponding button assignments.

At 916, the method 900 includes receiving, from one or more buttons of the instrument, an indication that one of the buttons was pressed by the user. Receiving the indication may include, for example, receiving an indication that the button was pressed, how long the button was pressed, etc.

At 920, the method 900 includes generating and outputting, in response to the indication that the button was pressed and based on an action assigned to the button that was pressed, a control signal. For example, for some device types, generating the control signal may include providing the control signal to a motor controller or other control circuitry internal to the instrument to control the device. Conversely, for other device types, generating the control signal may include providing the control signal to an external system, such as a surgical system. In examples where the control signal is provided to an external system, the control signal may simply indicate that the button was pressed or control functions or actions of the system in accordance with the action assigned to the button that was pressed.

At 924, the method 900 includes controlling or performing an action associated with the connected device in response to the button being pressed. Performing the action may include, but is not limited to, controlling the device connected the handpiece of the instrument (e.g., rotating a cutting tool, oscillating a cutting tool, etc.) or controlling the surgical system, an application or component of the surgical system, etc.

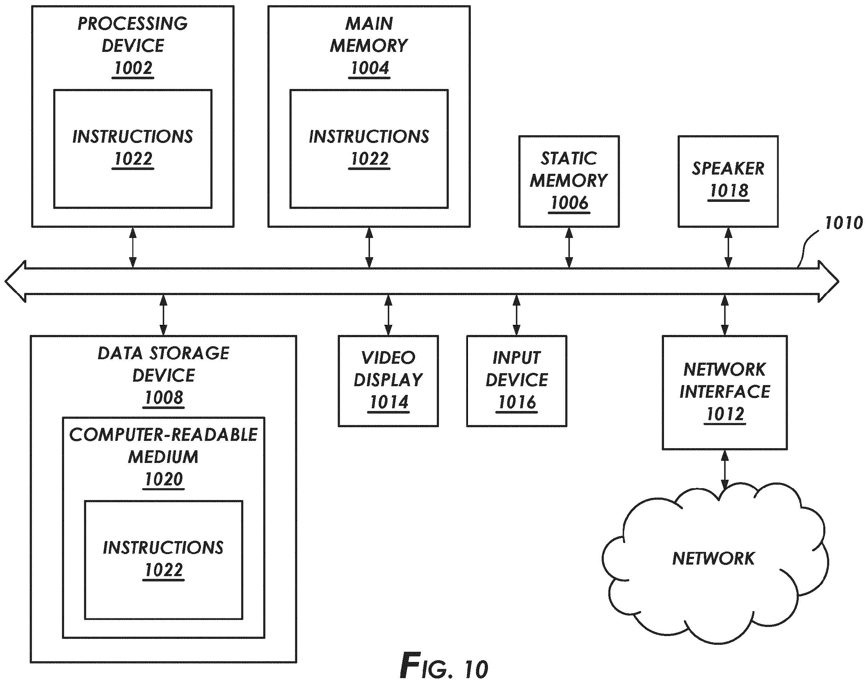

Figure 10 shows an example computer system or computing device 1000 configured to implement the various systems and methods of the present disclosure. In one example, the computer system 1000 may correspond to one or more computing devices of the system 100, the surgical controller 118, a device that creates a patient-specific instrument, a tablet device within the surgical room, the controller 812, or any other system that implements any or all the various methods discussed in this specification. For example, the computer system 1000 may be configured to implement all or portions of the method 600, the method 900, etc. The computer system 1000 may be connected (e.g., networked) to other computer systems in a local-area network (LAN), an intranet, and/or an extranet (e.g., device cart 102 network), or at certain times the Internet (e.g., when not in use in a surgical procedure). The computer system 1000 may be a server, a personal computer (PC), a tablet computer or any device capable of executing a set of instructions (sequential or otherwise) that specify actions to be taken by that device. Further, while only a single computer system is illustrated, the term "computer" shall also be taken to include any collection of computers that individually or jointly execute a set (or multiple sets) of instructions to perform any one or more of the methods discussed herein.

The computer system 1000 includes a processing device 1002, a main memory 1004 (e.g., read-only memory (ROM), flash memory, dynamic random access memory (DRAM) such as synchronous DRAM (SDRAM)), a static memory 1006 (e.g., flash memory, static random access memory (SRAM)), and a data storage device 1008, which communicate with each other via a bus 1010.

Processing device 1002 represents one or more general-purpose processing devices such as a microprocessor, central processing unit, or the like. More particularly, the processing device 1002 may be a complex instruction set computing (CISC) microprocessor, reduced instruction set computing (RISC) microprocessor, very long instruction word (VLIW) microprocessor, or a processor implementing other instruction sets or processors implementing a combination of instruction sets. The processing device 1002 may also be one or more special-purpose processing devices such as an application specific integrated circuit (ASIC), a field programmable gate array (FPGA), a digital signal processor (DSP), network processor, or the like. The processing device 1002 is configured to execute instructions for performing any of the operations and steps discussed herein. Once programmed with specific instructions, the processing device 1002, and thus the entire computer system 1000, becomes a special-purpose device, such as the surgical controller 118.

The computer system 1000 may further include a network interface device 1012 for communicating with any suitable network (e.g., the device cart 102 network). The computer system 1000 also may include a video display 1014 (e.g., the display device 114), one or more input devices 1016 (e.g., a microphone, a keyboard, and/or a mouse), and one or more speakers 1018. In one illustrative example, the video display 1014 and the input device(s) 1016 may be combined into a single component or device (e.g., an LCD touch screen).

The data storage device 1008 may include a computer-readable storage medium 1020 on which the instructions 1022 (e.g., implementing any methods and any functions performed by any device and/or component depicted described herein) embodying any one or more of the methodologies or functions described herein is stored. The instructions 1022 may also reside, completely or at least partially, within the main memory 1004 and/or within the processing device 1002 during execution thereof by the computer system 1000. As such, the main memory 1004 and the processing device 1002 also constitute computer-readable media. In certain cases, the instructions 1022 may further be transmitted or received over a network via the network interface device 1012.

While the computer-readable storage medium 1020 is shown in the illustrative examples to be a single medium, the term "computer-readable storage medium" should be taken to include a single medium or multiple media (e.g., a centralized or distributed database, and/or associated caches and servers) that store the one or more sets of instructions. The term "computer-readable storage medium" shall also be taken to include any medium that is capable of storing, encoding or carrying a set of instructions for execution by the machine and that cause the machine to perform any one or more of the methodologies of the present disclosure. The term "computer-readable storage medium" shall accordingly be taken to include, but not be limited to, solid-state memories, optical media, and magnetic media.

The foregoing description is merely illustrative in nature and is in no way intended to limit the disclosure, its application, or uses. The broad teachings of the disclosure can be implemented in a variety of forms. Therefore, while this disclosure includes particular examples, the true scope of the disclosure should not be so limited since other modifications will become apparent upon a study of the drawings, the specification, and the following claims. It should be understood that one or more steps within a method may be executed in different order (or concurrently) without altering the principles of the present disclosure. Further, although each of the embodiments is described above as having certain features, any one or more of those features described with respect to any embodiment of the disclosure can be implemented in and/or combined with features of any of the other embodiments, even if that combination is not explicitly described. In other words, the described embodiments are not mutually exclusive, and permutations of one or more embodiments with one another remain within the scope of this disclosure.

Spatial and functional relationships between elements (for example, between device, modules, circuit elements, etc.) are described using various terms, including "connected," "engaged," "coupled," "adjacent," "next to," "on top of," "above," "below," and "disposed." Unless explicitly described as being "direct," when a relationship between first and second elements is described in the above disclosure, that relationship can be a direct relationship where no other intervening elements are present between the first and second elements, but can also be an indirect relationship where one or more intervening elements are present (either spatially or functionally) between the first and second elements. Nevertheless, this paragraph shall serve as antecedent basis in the claims for referencing any electrical connection as "directly coupled" for electrical connections shown in the drawing with no intervening element(s).

As used herein, the phrase at least one of A, B, and C should be construed to mean a logical (A OR B OR C), using a non-exclusive logical OR, and should not be construed to mean "at least one of A, at least one of B, and at least one of C."

Broadly speaking, a controller, module, or circuitry may be defined as electronics having various integrated circuits, logic, memory, and/or software that receive instructions, issue instructions, control operation, enable cleaning operations, enable endpoint measurements, and the like. The integrated circuits may include chips in the form of firmware that store program instructions, digital signal processors (DSPs), chips defined as application specific integrated circuits (ASICs), and/or one or more microprocessors, or microcontrollers that execute program instructions (e.g., software). Program instructions may be instructions communicated to the controller in the form of various individual settings (or program files), defining operational parameters for carrying out a particular process.

Aspects of the disclosure are also found in the following numbered clauses:
1. A method for dynamically assigning actions to a plurality of input devices of a first surgical instrument connected to a surgical system, the method comprising, using one or more computing devices:
   with the surgical system in a first state, assigning a first action to a first input device of the plurality of input devices, wherein, in the first state, the surgical system is configured to cause the first action to be performed in response to the first input device being actuated;
   detecting a second surgical instrument; and
   in response to detecting the second surgical instrument, (i) transitioning the surgical system to a second state and (ii) assigning a second action to the first input device, wherein, in the second state, the surgical system is configured to cause the second action to be performed in response to the first input device being actuated.
2. The method of clause 1, wherein the first state and the second state correspond to a state of an application being executed using the surgical system.
3. The method of clause 1 or 2, wherein the first surgical instrument includes a camera head, and wherein the plurality of input devices includes a plurality of buttons arranged on the camera head.
4. The method of clause 1, 2 or 3, wherein the surgical system includes at least one of (i) a programmable footswitch with configurable buttons and (ii) a system configured to capture gestures as inputs.
5. The method of any of the previous clauses, wherein the second surgical instrument includes at least one of an aimer, an aligner, a probe, a grasper, a pincher, a scissor, a punch, a microfracture pick, a knife, a meniscectomy tool, a curette, an anchor deployment device, a biologic deployment device, a suture anchor deployment device, an arthroscopic blade, an arthroscopic RF wand, an arthroscopic coblation wand, a bone fiducial, and a fiducial on an instrument.
6. The method of any of the previous clauses, wherein the plurality of input devices includes a second input device, and wherein a third action assigned to the second input device is the same regardless of whether the surgical system is in the first state or the second state.
7. The method of any of the previous clauses, wherein detecting the second surgical instrument includes detecting the second surgical instrument using a camera.
8. The method of any of the previous clauses, further comprising transitioning the surgical system to the second state in response completion of a current action by one or more of the surgical system, the first surgical instrument, and the second surgical instrument.
9. The method of any of the previous clauses, further comprising detecting a bone fiducial prior to detecting the second surgical instrument, wherein the first surgical instrument is an arthroscopic instrument including a camera head, and wherein the bone fiducial is configured to be fixedly attached to patient anatomy.
10. The method of clause 9, further comprising (i) in response to detecting the bone fiducial, transitioning the surgical system to the first state and assigning the first action to the first input device and (ii) in response to detecting the second surgical instrument, transitioning the surgical system to the second state and assigning the second action to the first input device.
11. A processor configured to execute instructions stored in memory to dynamically assign actions to a plurality of input devices of a first surgical instrument connected to a surgical system, wherein executing the instructions causes the processor to:
   with the surgical system in a first state, assign a first action to a first input device of the plurality of input devices, wherein, in the first state, the surgical system is configured to cause the first action to be performed in response to the first input device being actuated;
   detect a second surgical instrument; and
   in response to detecting the second surgical instrument, (i) transition the surgical system to a second state and (ii) assign a second action to the first input device, wherein, in the second state, the surgical system is configured to cause the second action to be performed in response to the first input device being actuated.
12. The processor of clause 11, wherein the first state and the second state correspond to a state of an application being executed using the surgical system.
13. The processor of clause 11 or 12, wherein the first surgical instrument includes a camera head, and wherein the plurality of input devices includes a plurality of buttons arranged on the camera head.
14. The processor of clause 11, 12 or 13, wherein the processor is configured to receive inputs from at least one of (i) a programmable footswitch with configurable buttons and (ii) a system configured to capture gestures as the inputs.
15. The processor of any of clauses 11 to 14, wherein the second surgical instrument includes at least one of an aimer, an aligner, a probe, a grasper, a pincher, a scissor, a punch, a microfracture pick, a knife, a meniscectomy tool, a curette, an anchor deployment device, a biologic deployment device, a suture anchor deployment device, an arthroscopic blade, an arthroscopic RF wand, an arthroscopic coblation wand, a bone fiducial, and a fiducial on an instrument.
16. The processor of any of clauses 11 to 15, wherein the plurality of input devices includes a second input device, wherein a third action assigned to the second input device is the same regardless of whether the surgical system is in the first state or the second state.
17. The processor of any of clauses 11 to 16, wherein detecting the second surgical instrument includes detecting the second surgical instrument using a camera.
18. The processor of any of clauses 11 to 17, wherein executing the instructions further causes the processor to transition the surgical system to the second state in response completion of a current action by one or more of the surgical system, the first surgical instrument, and the second surgical instrument.
19. The processor of any of clauses 11 to 18, wherein executing the instructions further causes the processor to detect a bone fiducial prior to detecting the second surgical instrument, wherein the first surgical instrument is an arthroscopic instrument including a camera head, and wherein the bone fiducial is configured to be fixedly attached to patient anatomy.
20. The processor of clause 19, wherein executing the instructions further causes the processor to (i) in response to detecting the bone fiducial, transition the surgical system to the first state and assign the first action to the first input device and (ii) in response to detecting the second surgical instrument, transition the surgical system to the second state and assign the second action to the first input device.
21. A method for operating a surgical instrument coupled to a surgical system, the method comprising, using one or more computing devices:
   detecting, via a connection interface defined on a handpiece of the surgical instrument, a first device type of a first surgical device coupled to the handpiece;
   in response to detecting the first device type of the first surgical device, assigning first actions to respective input devices of a plurality of input devices arranged on the handpiece; and
   in response to actuation of one or more of the plurality of input devices, controlling at least one of the surgical instrument and the surgical system in accordance with the first actions assigned to the plurality of input devices.
22. The method of clause 21, wherein detecting the first device type includes at least one of (i) detecting the first device type based on an electrical signal or input data received from the first surgical device, (ii) detecting the first device type based on mechanical engagement between the first surgical device and the connection interface, and (iii) detecting a magnetic signal between the first surgical device and the connection interface.
23. The method of clause 21 or 22, wherein the first device type corresponds to a probe device configured to interact with an anatomical surface for a surgical operation.
24. The method of clause 21, 22 or 23, further comprising:
   detecting, via the connection interface defined on a handpiece of the surgical instrument, a second device type of a second surgical device coupled to the handpiece;
   in response to detecting the second device type of the second surgical device, assigning second actions to the respective input devices of the plurality of input devices arranged on the handpiece; and
   in response to actuation of the one or more of the plurality of input devices, controlling at least one of the surgical instrument and the surgical system in accordance with the second actions assigned to the plurality of input devices.
25. The method of clause 24, wherein the second device type corresponds to at least one of a shaver blade and a burr tool.
26. The method of clause 24 or 25, wherein the first actions correspond to registration functions for transmitting registration inputs from the surgical instrument to the surgical system and the second actions correspond to motor control functions for controlling a shaving or cutting operation of the surgical instrument.

## Claims

1. A processor configured to execute instructions stored in memory to dynamically assign actions to a plurality of input devices of a first surgical instrument connected to a surgical system, wherein executing the instructions causes the processor to:
with the surgical system in a first state, assign a first action to a first input device of the plurality of input devices, wherein, in the first state, the surgical system is configured to cause the first action to be performed in response to the first input device being actuated;
detect a second surgical instrument; and
in response to detecting the second surgical instrument, (i) transition the surgical system to a second state and (ii) assign a second action to the first input device, wherein, in the second state, the surgical system is configured to cause the second action to be performed in response to the first input device being actuated.

2. The processor of claim 1, wherein the first state and the second state correspond to a state of an application being executed using the surgical system.

3. The processor of claim 1 or 2, wherein the first surgical instrument includes a camera head, and wherein the plurality of input devices includes a plurality of buttons arranged on the camera head.

4. The processor of any of claims 1 to 3, wherein the processor is configured to receive inputs from at least one of (i) a programmable footswitch with configurable buttons and (ii) a system configured to capture gestures as the inputs.

5. The processor of any of claims 1 to 4, wherein the second surgical instrument includes at least one of an aimer, an aligner, a probe, a grasper, a pincher, a scissor, a punch, a microfracture pick, a knife, a meniscectomy tool, a curette, an anchor deployment device, a biologic deployment device, a suture anchor deployment device, an arthroscopic blade, an arthroscopic RF wand, an arthroscopic coblation wand, a bone fiducial, and a fiducial on an instrument.

6. The processor of any of claims 1 to 5, wherein the plurality of input devices includes a second input device, wherein a third action assigned to the second input device is the same regardless of whether the surgical system is in the first state or the second state.

7. The processor of any of claims 1 to 6, wherein detecting the second surgical instrument includes detecting the second surgical instrument using a camera.

8. The processor of any of claims 1 to 7, wherein executing the instructions further causes the processor to transition the surgical system to the second state in response completion of a current action by one or more of the surgical system, the first surgical instrument, and the second surgical instrument.

9. The processor of any of claims 1 to 8, wherein executing the instructions further causes the processor to detect a bone fiducial prior to detecting the second surgical instrument, wherein the first surgical instrument is an arthroscopic instrument including a camera head, and wherein the bone fiducial is configured to be fixedly attached to patient anatomy.

10. The processor of claim 9, wherein executing the instructions further causes the processor to (i) in response to detecting the bone fiducial, transition the surgical system to the first state and assign the first action to the first input device and (ii) in response to detecting the second surgical instrument, transition the surgical system to the second state and assign the second action to the first input device.
